# EUROPEAN PATENT SPECIFICATION

(11) **EP 2 349 275 B1**
(45) Date of publication and mention of the grant of the patent: **08.03.2017**
(21) Application number: 09744391.5
(22) Date of filing: 29.10.2009
(51) Int. Cl.: A61K 31/5377, A61P 35/00, A61K 45/06, A61K 31/436

(54) **COMBINATION OF A PHOSPHATIDYLINOSITOL-3-KINASE (PI3K) INHIBITOR AND A MTOR INHIBITOR.**
KOMBINATION EINES PHOSPHOINOSITID-3-KINASE-(PI3K)-HEMMERS UND EINES MTOR-HEMMERS.
COMBINAISON D'UN INHIBITEUR DE LA PHOSPHOINOSITIDE 3-KINASE (PI3K) ET DE LA MTOR.

(30) Priority: 31.10.2008 EP 08168044
(43) Date of publication of application: 03.08.2011
(73) Proprietor: Novartis AG, 4056 Basel (CH)
(72) Inventor: GARCIA-ECHEVERRIA, Carlos, CH-4002 Basel (CH); MAIRA, Sauveur-Michel, CH-4002 Basel (CH)
(74) Representative: Gruber, Markus
(86) International application number: PCT/EP2009/064274
(87) International publication number: WO 2010/049481

(56) References cited:
- WO-A-2006/044453
- WO-A-2007/084786
- WO-A-2007/120897
- WO-A-2008/098058
- WINBANKS ET AL: "Role of the phosphatidylinositol 3-kinase and mTOR pathways in the regulation of renal fibroblast function and differentiation" INTERNATIONAL JOURNAL OF BIOCHEMISTRY AND CELL BIOLOGY, EXETER, GB, vol. 39, no. 1, 1 January 2007 (2007-01-01), pages 206-219, XP005708217 ISSN: 1357-2725
- SUN SHI-YONG ET AL: "Activation of Akt and eIF4E survival pathways by rapamycin-mediated mammalian target of rapamycin inhibition" CANCER RESEARCH, vol. 65, no. 16, August 2005 (2005-08), pages 7052-7058, XP002514945 ISSN: 0008-5472
- CHAISUPARAT RISA ET AL: "Dual inhibition of PI3Kalpha and mTOR as an alternative treatment for Kaposi's sarcoma." CANCER RESEARCH 15 OCT 2008, vol. 68, no. 20, 15 October 2008 (2008-10-15), pages 8361-8368, XP002514946 ISSN: 1538-7445
- X. Wang ET AL: "Enhancing Mammalian Target of Rapamycin (mTOR)-Targeted Cancer Therapy by Preventing mTOR/Raptor Inhibition-Initiated, mTOR/Rictor-Independent Akt Activation", Cancer research, vol. 68, no. 18, 15 September 2008 (2008-09-15), pages 7409-7418, XP055142072, ISSN: 0008-5472, DOI: 10.1158/0008-5472.CAN-08-1522
- XUERONG WANG ET AL: "Enhancing mTOR-targeted cancer therapy", EXPERT OPINION ON THERAPEUTIC TARGETS, ASHLEY PUBLICATIONS, LONDON, GB, vol. 13, no. 10, 1 October 2009 (2009-10-01), pages 1193-1203, XP009180336, ISSN: 1472-8222, DOI: 10.1517/14728220903225008
- Hui Ren ET AL: "The combination of RAD001 and NVP-BKM120 synergistically inhibits the growth of lung cancer in vitro and in vivo", Cancer Letters, vol. 325, no. 2, 1 December 2012 (2012-12-01), pages 139-146, XP055142043, ISSN: 0304-3835, DOI: 10.1016/j.canlet.2012.06.018

## Description

### Field of the Invention

The present invention relates to a pharmaceutical combination comprising a phosphatidylinositol- 3-kinase (PI3K) inhibitor compound which is the compound 5-(2,6-dimorpholin-4-yl-pyrimidin-4-yl)-4-trifluoromethyl-pyridin-2-ylamine, or a stereoisomer, tautomer, or pharmaceutically acceptable salt thereof and a mTOR inhibitor which is everolimus, and the uses of such a combination in the treatment of proliferative diseases, more specifically of mammalian target of rapamycin (mTOR) kinase dependent diseases, as described in claims.

### Background of the Invention

It has been shown that mTOR inhibition can induce upstream insulin-like growth factor 1 receptor (IGF-1 R) signaling resulting in AKT activation in cancer cells. This phenomenon has been suggested to play a role in the attenuation of cellular responses to mTOR inhibition and may attenuate the clinical activity of mTOR inhibitors. Increase in pAKT has for instance been found in approximately 50% in the tumours of all patients in a Phase I study in patients with advanced solid tumours (Taberno et al., Journal of Clinical Oncology, 26 (2008), pp 1603-1610). In spite of numerous treatment options for proliferative disease patients, there remains a need for effective and safe therapeutic agents and a need for their preferential use in combination therapy.

WO 2008/098058 discloses certain pyrimidine derivatives as PI3K inhibitor compounds and methods of their use either alone or in combination with at least one additional therapeutic agent.

WO 2006/044453 discloses analogs of 17-hydroxywortmannin as PI3K inhibitors and methods of their use.

WO 2007/120897 discloses compositions containing rapamycin or rapamycin derivatives in combination with a PI3 kinase inhibitor and/or a leptin inhibitor, and methods for the treatment and/or prevention of intimal hyperplasia, vascular stenosis and/or restenosis.

Winbanks et al., "Role of the phosphatidylinositol 3-kinase and mTOR pathways in the regulation of renal fibroblast function and differentiation", Internal Journal of Biochemistry and Cell Biology, EXETER, GB, vol. 39, no. 1, 1 January 2007, pp. 206-219] relates to specific inhibitors of mTOR (RAD) and phosphatidylinositol 3-kinase (LY294002) and the mechanisms of fibrogenesis.

Sun Shi-Yong et al., "Activation of Akt and eIF4E survival pathways by rapamycin-mediated mammalian target of rapamycin inhibition", Cancer Research, vol. 65, no. 16, August 2005 (2005-08), pp. 7052-7058] discloses the effects of LY294002 in combination with rapamycin on human NSCLC cells.

Chaisuparat Risa et al., ["Dual inhibition of PI3Kalpha and mTOR as an alternative treatment for Kaposi's sarcoma", Cancer Research, 15 OCT 2008, val. 68, no. 20, 15 October 2008 (2008-1 0-15), pp. 8361-8368] discusses PI-103 as a therapeutic option for the treatment of Kaposi's sarcoma.

### Summary of the Invention

It has been now been found in accordance with the present disclosure that a PI3K inhibitor reduces or blocks the phosphorylation and activation of AKT by mTOR inhibitors. Accordingly, the present disclosure provides a method to reduce or block the phosphorylation and activation of AKT by mTOR inhibitors comprising administering a PI3K inhibitor to a warm-blooded animal in need thereof.

The present invention provides a pharmaceutical combination comprising (a) the compound 5-(2,6-di-morpholin-4-yl-pyrimidin-4-yl)-4-trifluoromethyl-pyridin-2-ylamine, or a stereoisomer, tautomer, or pharmaceutically acceptable salt thereof, and (b) a mTOR inhibitor which is everolimus.

In another embodiment, the present disclosure provides a method of treating a proliferative disease dependent on acquired phosphorylation and activation of AKT during treatment with an mTOR inhibitor comprising administering a therapeutically effective amount of a PI3K inhibitor to a warm-blooded animal in need thereof.

In another embodiment, the present disclosure relates to a method of treating a proliferative disease which has become resistant or has a decreased sensitivity to the treatment with an mTOR inhibitor comprising administering a therapeutically effective amount of a PI3K inhibitor to a warm-blooded animal in need thereof. The resistance is e.g. due to posphorylation and activation of AKT.

In a further aspect the present disclosure provides a method for improving efficacy of the treatment of a proliferative disease with an mTOR inhibitor comprising administering a combination comprising a PI3K inhibitor and a mTOR inhibitor to a warm-blooded animal in need thereof.

In one aspect the present disclosure provides a pharmaceutical composition comprising a PI3K inhibitor compound and at least one mTOR inhibitor.

In another aspect the present disclosure provides the use of a PI3K inhibitor compound and at least one mTOR inhibitor for the manufacture of a medicament for the treatment or prevention of a proliferative disease.

In another aspect the present disclosure provides a method of treating or preventing a proliferative by administering a PI3K inhibitor compound and at least one mTOR inhibitor.

In another aspect the present invention provides a pharmaceutical combination comprising a PI3K inhibitor compound 5-(2,6-di-morpholin-4-yl-pyrimidin-4-yl)-4-trifluoromethyl-pyridin-2-ylamine, or a stereoisomer, tautomer, or pharmaceutically acceptable salt thereof and at least one mTOR inhibitor which is everolimus for use in treating or preventing of a mammalian target of rapamycin (mTOR) kinase dependent disease which is selected from breast cancer, renal cell carcinoma, gastric tumors, neuroendocrine tumors, lymphomas and prostate cancer. In one embodiment of the present invention, each combination partner of the combination is administered simultaneously or sequentially and in any order.

In one aspect the present invention provides a pharmaceutical composition comprising a PI3K inhibitor compound 5-(2,6-di-morpholin-4-yl-pyrimidin-4-yl)-4-trifluoromethyl-pyridin-2-ylamine, or a stereoisomer, tautomer, or pharmaceutically acceptable salt thereof and a mTOR inhibitor which is everolimus.

### Detailed Description of the Figures

Figure 1 shows the AKT phosphorylation levels in presence of everolimus (RAD001) and everolimus (RAD001) in combination with Compound I in BT474 breast tumor cells.
Figure 2 shows the AKT phosphorylation levels in presence of everolimus (RAD001) and everolimus (RAD001) in combination with Compound I in MDA-MB-231 breast tumor cells.

### Detailed Description of the Invention

WO07/084786 describes pyrimidine derivatives, which have been found the activity of lipid kinases, such as PI3-kinases.

A preferred compound of the present inventive combination is a compound which is specifically described in WO07/084786 and which is 5-(2,6-di-morpholin-4-yl-pyrimidin-4-yl)-4-trifluoromethyl-pyridin-2-ylamine (Compound I). The synthesis of 5-(2,6-di-morpholin-4-yl-pyrimidin-4-yl)-4-trifluoromethyl-pyridin-2-ylamine is described in WO07/084786 as Example 10.

Combinations of the present disclosure include compounds which target, decrease or inhibit the activity/function of serine/theronine mTOR kinase. Such compounds will be referred to as "mTOR inhibitors" and include but is not limited to compounds, proteins or antibodies which target/inhibit members of the mTOR kinase family, e.g., RAD, rapamycin (sirolimus) and derivatives/analogs thereof such as everolimus or RAD001 or compounds that inhibit the kinase activity of mTOR by directly binding to the ATP-binding cleft of the enzyme. Sirolimus is also known by the name RAPAMUNE and everolimus or RAD001 by the name CERTICAN. Other compounds, proteins or antibodies which target/inhibit members of the mTOR kinase family include CCI-779, ABT578, SAR543, and ascomycin which is an ethyl analog of FK506. Also included are AP23573, AP23841, KU-0063794, INK-128, EX2044, EX3855, EX7518, AZD08055 and OSI027. A particularly preferred compound in accordance with the present invention is RAD001.

Suitable mTOR inhibitors include e.g.:
I. Rapamycin which is an immunosuppressive lactam macrolide that is produced by Streptomyces hyGroscopicus.
II. Rapamycin derivatives such as:
a. substituted rapamycin e.g. a 40-O-substituted rapamycin e.g. as described in US 5,258,389, WO 94/09010, WO 92/05179, US 5,118,677, US 5,118,678, US 5,100,883, US 5,151,413, US 5,120,842, WO 93/11130, WO 94/02136, WO 94/02485 and WO 95/14023;
b. a 16-O-substituted rapamycin e.g. as disclosed in WO 94/02136, WO 95/16691 and WO 96/41807;
c. a 32-hydrogenated rapamycin e.g. as described in WO 96/41807 and US 5 256 790.
d. Preferred rapamycin derivatives are compounds of formula (II)

| | |
|---|---|
| 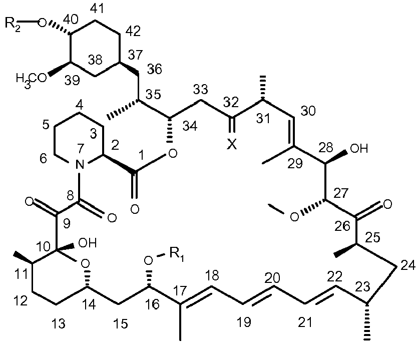 | II |

wherein
R₁ is CH₃ or C₃₋₆alkynyl,
R₂ is H or -CH₂-CH₂-OH, 3-hydroxy-2-(hydroxymethyl)-2-methyl-propanoyl or tetrazolyl, and X is =O, (H,H) or (H,OH)
provided that R₂ is other than H when X is =O and R₁ is CH₃,
or a prodrug thereof when R₂ is -CH₂-CH₂-OH, e.g. a physiologically hydrolysable ether thereof.

Compounds of formula (II) are disclosed e.g. in WO 94/09010, WO 95/16691 or WO 96/41807. They may be prepared as disclosed or by analogy to the procedures described in these references.

Suitable compounds are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-0-(2-hydroxyethyl)-rapamycin, disclosed as Example 8 in WO 94/09010.

Particularly suitable rapamycin derivatives of formula (II) are 40-O-(2-hydroxyethyl)-rapamycin, 40-[3-hydroxy-2-(hydroxymethyl)-2-methylpropanoate]-rapamycin (also called CCI779), 40-epi-(tetrazolyl)-rapamycin (also called ABT578), 32-deoxorapamycin, 16-pent-2-ynyloxy-32(S)-dihydro rapamycin, or TAFA-93.
e. Rapamycin derivatives also include so-called rapalogs, e.g. as disclosed in WO 98/02441 and WO 01/14387, e.g. AP23573, AP23464, or AP23841.

Rapamycin and derivatives thereof have, on the basis of observed activity, e.g. binding to macrophilin-12 (also known as FK-506 binding protein or FKBP-12), e.g. as described in WO 94/09010, WO 95/16691 or WO 96/41807, been found to be useful e.g. as immuno-suppressant, e.g. in the treatment of acute allograft rejection.
III. Ascomycin, which is an ethyl analog of FK506.
IV AZD08055 and OS1127, which are compounds that inhibit the kinase activity of mTOR by directly binding to the ATP-binding cleft of the enzyme

Comprised are likewise the pharmaceutically acceptable salts thereof, the corresponding racemates, diastereoisomers, enantiomers, tautomers, as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e., a pharmaceutical combination within the scope of this invention could include three active ingredients or more.

In one aspect the present invention provides a pharmaceutical composition comprising a PI3K inhibitor compound 5-(2,6-di-morpholin-4-yl-pyrimidin-4-yl)-4-trifluoromethyl-pyridin-2-ylamine as described above and at least one mTOR inhibitor which is everolimus.

In another aspect there is also provided the use of a PI3K inhibitor compound 5-(2,6-di-morpholin-4-yl-pyrimidin-4-yl)-4-trifluoromethyl-pyridin-2-ylamine and at least one mTOR inhibitor for the manufacture of a medicament for the treatment or prevention of a proliferative disease.

In a further aspect the present disclosure provides a compound 5-(2,6-di-morpholin-4-yl-pyrimidin-4-yl)-4-trifluoromethyl-pyridin-2-ylamine and at least one mTOR inhibitor for use in treating or preventing a proliferative disease.

In another aspect the present disclosure provides a method of treating or preventing a proliferative disease by administering a compound 5-(2,6-di-morpholin-4-yl-pyrimidin-4-yl)-4-trifluoromethyl-pyridin-2-ylamine and at least one mTOR inhibitor.

In another aspect the present disclosure provides a pharmaceutical combination comprising a compound 5-(2,6-di-morpholin-4-yl-pyrimidin-4-yl)-4-trifluoromethyl-pyridin-2-ylamine and at least one mTOR inhibitor for use in treating or preventing a proliferative disease.

In another aspect the present disclosure provides a combination of a compound 5-(2,6-di-morpholin-4-yl-pyrimidin-4-yl)-4-trifluoromethyl-pyridin-2-ylamine (Compound I) and an mTOR inhibitor selected from the group consisting of RAD rapamycin (sirolimus) and derivatives/analogs thereof such as everolimus or RAD001; CCI-779, ABT578, SAR543, ascomycin (an ethyl analog of FK506), AP23573, AP23841, KU-0063794, INK-128, EX2044, EX3855, EX7518, AZD08055 and OSI027, wherein the active ingredients are present in each case in free form or in the form of a pharmaceutically acceptable salt, and optionally at least one pharmaceutically acceptable carrier, for simultaneous, separate or sequential use for the treatment of mammalian target of rapamycin (mTOR) kinase dependent diseases.

The present disclosure provides a method to reduce or block the phosphorylation and activation of AKT by mTOR inhibitors comprising administering a compound of formula (II) to a warm-blooded animal in need thereof. In another embodiment, the present disclosure provides a method of treating a proliferative disease dependent on acquired phosphorylation and activation of AKT during treatment with an mTOR inhibitor comprising administering a therapeutically effective amount of a compound (I) to a warm-blooded animal in need thereof.

In another embodiment, the present disclosure relates to a method of treating a proliferative disease which has become resistant or has a decreased sensitivity to the treatment with an mTOR inhibitor comprising administering a therapeutically effective amount of a compound (I) to a warm-blooded animal in need thereof. The resistance is e.g. due to posphorylation and activation of AKT.

In a further aspect the present disclosure provides a method for improving efficacy of the treatment of a proliferative disease with an mTOR inhibitor comprising administering a combination comprising a compound 5-(2,6-di-morpholin-4-yl-pyrimidin-4-yl)-4-trifluoromethyl-pyridin-2-ylamine and a mTOR inhibitor to a warm-blooded animal in need thereof.

The mTOR inhibitor used according to the present disclosure may be selected from RAD rapamycin (sirolimus) and derivatives/analogs thereof such as everolimus or RAD001; CCI-779, ABT578, SAR543, ascomycin (an ethyl analog of FK506), AP23573, AP23841, KU-0063794, INK-128, EX2044, EX3855, EX7518, AZD08055 and OSIO27. Particularly preferred mTOR inhibitors in accordance with the present disclosure are sirolimus and/or everolimus.

The term "mTOR kinase dependent diseases" includes but is not restricted to the following symptoms:
- Organ or tissue transplant rejection, e.g. for the treatment of recipients of e.g. heart, lung, combined heart-lung, liver, kidney, pancreatic, skin or corneal transplants; graft-versus-host disease, such as following bone marrow transplantation;
- Restenosis
- Hamartoma syndromes, such as tuberous sclerosis or Ccowden Disease
- Lymphangioleiomyomatosis
- Retinitis pigmentosis
- Autoimmune diseases including encephalomyelitis, insulin-dependent diabetes mellitus, lupus, dermatomyositis, arthritis and rheumatic diseases
- Steroid-resistant acute Lymphoblastic Leukaemia
- Fibrotic diseases including scleroderma, pulmonary fibrosis, renal fibrosis, cystic fibrosis
- Pulmonary hypertension
- Immunomodulation
- Multiple sclerosis
- VHL syndrome
- Carney complex
- Familial adenonamtous polyposis
- Juvenile polyposis syndrome
- Birt-Hogg-Duke syndrome
- Familial hypertrophic cardiomyopathy
- Wolf-Parkinson-White syndrome
- Neurodegenarative disorders such as Parkinson's, Huntingtin's, Alzheimer's and dementias caused by tau mutations, spinocerebellar ataxia type 3, motor neuron disease caused by SOD1 mutations, neuronal ceroid lipofucinoses/Batten disease (pediatric neurodegeneration)
- wet and dry macular degeneration
- muscle wasting (atrophy, cachexia) and myopathies such as Danon's disease.
- bacterial and viral infections including M. tuberculosis, group A streptococcus, HSV type I, HIV infection
- Neurofibromatosis including Neurofibromatosis type 1,
- Peutz-Jeghers syndrome

Furthermore, "mTOR kinase dependent diseases" include cancers and other related malignancies. A non-limiting list of the cancers associated with pathological mTOR signaling cascades includes breast cancer, renal cell carcinoma, gastric tumors, neuroendocrine tumors, lymphomas and prostate cancer.

Examples for a proliferative disease are for instance benign or malignant tumor, carcinoma of the brain, kidney, liver, adrenal gland, bladder, breast, stomach, gastric tumors, ovaries, colon, rectum, prostate, pancreas, lung, vagina or thyroid, sarcoma, glioblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, lymphomas, a mammary carcinoma or a leukemia.

The pharmaceutical compositions or combination in accordance with the present invention can be tested in clinical studies. Suitable clinical studies may be, for example, open label, dose escalation studies in patients with proliferative diseases. Such studies prove in particular the synergism of the active ingredients of the combination of the invention. The beneficial effects on proliferative diseases may be determined directly through the results of these studies which are known as such to a person skilled in the art. Such studies may be, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) may be administered in a fixed dose and the dose of agent (b) may be escalated. Each patient may receive doses of the agent (a) either daily or intermittent. The efficacy of the treatment may be determined in such studies, e.g., after 12, 18 or 24 weeks by evaluation of symptom scores every 6 weeks.

The administration of a pharmaceutical combination of the invention may result not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit may be that lower doses of the active ingredients of the combination of the invention may be used, for example, that the dosages need not only often be smaller but may also be applied less frequently, which may diminish the incidence or severity of side-effects. This is in accordance with the desires and requirements of the patients to be treated.

It is one objective of this invention to provide a pharmaceutical composition comprising a quantity, which may be jointly therapeutically effective at targeting or preventing proliferative diseases a combination of the invention. In this composition, agent (a) and agent (b) may be administered together, one after the other or separately in one combined unit dosage form or in two separate unit dosage forms. The unit dosage form may also be a fixed combination.

The pharmaceutical compositions for separate administration of agent (a) and agent (b) or for the administration in a fixed combination, i.e. a single galenical composition comprising at least two combination partners (a) and (b), according to the invention may be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions may contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount may be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of preventing or treating proliferative diseases according to the invention may comprise (i) administration of the first agent (a) in free or pharmaceutically acceptable salt form and (ii) administration of an agent (b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert in vivo to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

### Example 1: Effect of the combination of RAD001 (everolimus) with Compound I in BT474 and MDA-MB-231 breast tumor cells

### Material and Methods

### 1. Preparation of compounds

The compound RAD001 is synthesized by Novartis Pharma AG. A 20 mM stock solution is prepared in DMSO and stored -20 °C. A 10 mM stock solution of the Compound I is prepared in DMSO and stored at -20 °C.

### 2. Cells and cell culture conditions

Human breast carcinoma BT474 (ATCC HTB-26) and MDA-MB-231 (ATCC HTB-20) are obtained from the American Type Culture Collection (ATCC, Rockville, MD, USA). BT474 cells are maintained in Hybri-Care medium (ATCC) supplemented with 10 % v/v fetal calf serum and 2 mM L-glutamine. MDA-MB-231 cells are grown in RPMI 1640 medium (Amimed, Allschwil, Switzerland) supplemented with 10 % v/v fetal calf serum and 2 mM L-glutamine. All media are supplemented with 100 *g/mL penicillin/streptomycin and cells are maintained at 37°C in 5 % CO2.

### 3. Cell treatment and cell extraction

BT474 and MDA-MB-231 cells are seeded at a density of 3.3X10⁴ cells/cm² and 1.6X10⁴ cells/cm², respectively, and incubated for 48 h at 37°C and 5 % CO₂, prior to treatment with DMSO vehicle, 20 nM RAD001 and/or various concentrations of Compound I for 24 h. Cell lysates are prepared as follows. Culture plates are washed once with ice-cold PBS containing 1mM PMSF and once with ice-cold extraction buffer [50 mM Hepes (pH 7.4), 150 mM NaCl, 25 mM β-glycerophosphate, 25 mM NaF, 5 mM EGTA, 1 mM EDTA, 15 mM PPi, 2 mM sodium orthovanadate, 10 mM sodium molybdate, leupeptin (10 µg/mL), aprotinin (10 µg/mL), 1 mM DTT and 1 mM PMSF]. Protease inhibitors are purchased from SIGMA Chemical, St. Louis, Mo. Cells are extracted in the same buffer, containing 1 % NP-40 (SIGMA Chemicals). The extracts re homogenized, cleared by centrifugation, aliquoted and frozen at -80°C. Protein concentration are determined with the BCA Protein Assay (Pierce, Rockford, IL, USA).

### 4. Immunoblotting

Twenty micrograms of cell extracts are resolved electrophoretically on 12% denaturing sodium dodecyl sulfate polyacrylamide gels (SDS-PAGE) and transferred to polyvinylidene difluoride filters (PVDF; Millipore Corporation, Bedford, MA, USA) by wet-blotting (1 h at 250 mA) and probed overnight at 4°C with the following primary antibodies:
anti-phospho-Akt (Ser473) (clone 14-05; 1:2000) obtained from DAKO (Glostrup, Denmark) and diluted in PBS, 0.5 % v/v Tween.
anti-phospho-Akt (T308) (cat # 9275; 1:1000) obtained from Cell Signaling Technology (Beverly, MA, USA) and diluted in PBS, 0.1 % v/v Tween.
anti-Akt (cat # 1085-1; 1:5000) obtained from Epitomics (Burlingame, CA, USA) and diluted in PBS, 0.5 % v/v Tween.
Anti-Actin (cat # MAB1501; 1:20,000) obtained from Chemicon (Billerica, MA, USA) and diluted in PBS, 0.1 % v/v Tween.

After incubation with the appropriate primary antibody (above), decorated proteins are revealed using horseradish peroxidase-conjugated anti-mouse or anti-rabbit immunoglobulins followed by enhanced chemiluminescence (ECL Plus kit; Amersham Pharmacia Biotech, Buckinghamshire, UK) and quantified using Quantity One Software (Bio-Rad, Munich, Germany).

## Claims

1. A pharmaceutical combination comprising
a) the compound 5-(2,6-di-morpholin-4-yl-pyrimidin-4-yl)-4-trifluoromethyl-pyridin-2-ylamine , or a stereoisomer, tautomer, or pharmaceutically acceptable salt thereof,
and
b) a mTOR inhibitor which is everolimus.

2. A pharmaceutical combination according to claim 1 for use in the treatment or prevention of a mammalian target of rapamycin (mTOR) kinase dependent disease which is selected from breast cancer, renal cell carcinoma, gastric tumors, neuroendocrine tumors, lymphomas and prostate cancer.

3. A pharmaceutical combination according to claim 1 for use according to claim 2 wherein each combination partner of the combination according to claim 1 is administered simultaneously or sequentially and in any order.

4. A pharmaceutical composition comprising the pharmaceutical combination according to claim 1.

## Patentansprüche

1. Pharmazeutische Kombination, die Folgendes umfasst:
a) die Verbindung 5-(2,6-Dimorpholin-4-ylpyrimidin-4-yl)-4-trifluormethylpyridin-2-ylamin, oder ein Stereoisomer, Tautomer oder pharmazeutisch unbedenkliches Salz davon,
und
b) einen mTOR-Hemmer, bei dem es sich um Everolimus handelt.

2. Pharmazeutische Kombination nach Anspruch 1 zur Verwendung in der Behandlung oder Vorbeugung einer von der "Mammalian Target of Rapamycin"(mTOR)-Kinase abhängigen Erkrankung, die aus Brustkrebs, Nierenzellenkarzinom, Magentumoren, neuroendokrinen Tumoren, Lymphomen und Prostatakrebs ausgewählt ist.

3. Pharmazeutische Kombination nach Anspruch 1 zur Verwendung nach Anspruch 2, wobei jeder Kombinationspartner der Kombination nach Anspruch 1 gleichzeitig oder nacheinander und in einer beliebigen Reihenfolge verabreicht wird.

4. Pharmazeutische Zusammensetzung, die die pharmazeutische Kombination nach Anspruch 1 umfasst.

## Revendications

1. Combinaison pharmaceutique comprenant
a) le composé 5-(2,6-di-morpholin-4-yl-pyrimidin-4-yl)-4-trifluorométhyl-pyridin-2-ylamine, ou un stéréoisomère, un tautomère, ou un sel pharmaceutiquement acceptable de celui-ci,
et
b) un inhibiteur de mTOR qui est l'évérolimus.

2. Combinaison pharmaceutique selon la revendication 1 pour utilisation dans le traitement ou la prévention d'une maladie dépendante de la kinase mTOR (cible de la rapamycine chez les mammifères) qui est choisie parmi le cancer du sein, un carcinome à cellules rénales, des tumeurs gastriques, des tumeurs neuroendocrines, des lymphomes et le cancer de la prostate.

3. Combinaison pharmaceutique selon la revendication 1 pour utilisation selon la revendication 2 dans laquelle chaque partenaire de combinaison de la combinaison selon la revendication 1 est administré simultanément ou séquentiellement et dans un ordre quelconque.

4. Composition pharmaceutique comprenant la combinaison pharmaceutique selon la revendication 1.
